# EUROPEAN PATENT APPLICATION

(11) **EP 1 733 721 A2**
(43) Date of publication of application: **20.12.2006**
(21) Application number: 06253086.0
(22) Date of filing: 14.06.2006
(51) Int. Cl.: A61K 31/122, A61K 31/23, A61K 31/231, A61P 11/00, A61P 11/06, A61P 9/10, A61P 9/00, A61P 29/00, A61P 37/00, A61P 7/02, A61P 25/00, A61P 3/06, A61P 3/04

(54) **Use of astaxanthin or esters thereof as a phosphodiesterase inhibitor**

(30) Priority: 15.06.2005 JP 2005174749; 17.10.2005 JP 2005301153
(71) Applicant: YAMAHA HATSUDOKI KABUSHIKI KAISHA, Iwata-shi, Shizuoka 438-8501 (JP)
(72) Inventor: Okada, Yumika c/o Yamaha Hatsudoki K. K., Iwata-shi, Shizuoka 438-8501 (JP)
(74) Representative: Schlich, George William

(57) **Abstract**

A PDE inhibitor and a therapeutic agent for a PDE-related disease of the present invention contain astaxanthin and/or esters thereof. The PDE inhibitor of the present invention is useful for treating various diseases exhibiting a relationship to PDEs, e.g., chronic obstructive pulmonary diseases, asthma, chronic artery obstruction, cardiovascular diseases, inflammatory diseases, allergic diseases, thrombosis, encephalopathy, hyperlipemia, and obesity.

## Description

### BACKGROUND OF THE INVENTION

### 1. Field of the Invention

The present invention relates to a phosphodiesterase inhibitor. More specifically, the present invention relates to a phosphodiesterase inhibitor and a therapeutic agent for a phosphodiesterase-related disease that contain astaxanthin and/or an ester thereof.

### 2. Description of the Related Art

Phosphodiesterases (PDEs) are enzymes that catalyze the hydrolysis of various cyclic nucleotide monophosphates (including cAMP and cGMP). cAMP and cGMP, which are intracellular transmitters, are metabolized by PDEs into inactive 5'-AMP and 5'-GMP, respectively. Such PDE isozymes are not uniformly present in the body, but rather there are differences in their cellular distribution and their histological distribution. That is, PDE isozymes are present in a body with a specific localization in an organ. It has been confirmed that there are 11 families of PDEs, namely PDE1 to PDE 11 (Current Opinion in Cell Biology., vol. 12, 2000, pp. 174-179). For example, PDE4 is present in various cells such as airway smooth muscles, epithelial cells, inflammatory cells (for example, macrophages, neutrophils, eosinophils), and T lymphocytes, and regulates the intracellular cAMP level of these cells to control the cell function. On the other hand, other PDEs such as PDE3 are present in blood platelets, cardiac muscles, vascular smooth muscles, and other tissues, and regulate the intracellular cGMP or cAMP level and are involved in control of the circulatory system. For example, PDE2 is cGMP-dependent and found in the heart and adrenal gland. PDE3 is inhibited by cGMP, and the inhibition of PDE3 causes positive muscular contraction activity. PDE5 regulates cGMP in various vascular smooth muscles. In this manner, PDE isozymes have different physiological functions from each other.

Thus, numerous medicaments for treating diseases related to PDE isozymes by inhibiting the respective PDE isozymes have been developed and put to practical use. For example, a PDE3 inhibitor is useful for treatment of cardiomyopathy, pulmonary hypertension, and associated pathological conditions, and has a blood platelet aggregation inhibitory ability and a lipolysis enhancing ability (Goodman Gilman's: The Pharmacological Basis of Therapeutics, Ninth Edition, pp. 832-837, McGraw-Hill, 1996; and Hiroyoshi Hidaka et al., Junkankika (Cardioangiology), vol. 37, 1995, pp. 232-242). A PDE4 inhibitor has a bronchodilator ability, an anti-inflammatory ability, an ability to inhibit mediator release, and an immunosuppressive ability. In recent years, it was found that a PDE5 inhibitor is effective for treating male erectile dysfunction (MED) and female sexual dysfunction (FSD).

As described above, most of the currently used PDE inhibitors are chemically synthesized substances, and PDE inhibitors derived from naturally-occurring products have not been studied very much. For example, although it has been reported that extracts from various naturally-occurring products have a PDE inhibitory ability (e.g., Japanese Laid-Open Patent Publication Nos. 2004-331648, 2004-26719, 2003-261457, 2002-87974, and 11-209299), the active components of those extracts have not yet been identified.

### SUMMARY OF THE INVENTION

It is an object of the present invention to provide a more inexpensive and safer PDE inhibitor derived from a naturally-occurring product.

The present invention provides a phosphodiesterase (PDE) inhibitor containing at least one of astaxanthin and an ester thereof.

The present invention also provides a therapeutic agent for a phosphodiesterase-related disease, which contains at least one of astaxanthin and an ester thereof.

In one embodiment, the disease is a chronic obstructive pulmonary disease, asthma, chronic artery obstruction, a cardiovascular disease, an inflammatory disease, an allergic disease, thrombosis, encephalopathy, hyperlipemia, or obesity.

Moreover, the present invention provides a method for inhibiting the activity of a phosphodiesterase, said method comprising bringing at least one member selected from the group consisting of astaxanthin and esters thereof in contact with a phosphodiesterase. The method can be carried out in vitro. The method may be a cosmetic method.

In addition, the present invention provides a method for treating a phosphodiesterase-related disease, for example a disease which can be treated using a phosphodiesterase inhibitor, said method comprising administering a therapeutically-effective amount of at least one member selected from the group consisting of astaxanthin and esters thereof to an individual suffering from the phosphodiesterase-related disease. The invention also provides use of astaxanthin or an ester thereof in manufacture of a medicament for treating a phosphodiesterase-related disease, e.g. a disease which can be treated using a phosphodiesterase inhibitor.

According to the present invention, a novel PDE inhibitor derived from a naturally-occurring product is provided. This PDE inhibitor can be used as therapeutic agents for various diseases exhibiting a relationship with various PDEs, such as chronic obstructive pulmonary diseases, asthma, chronic artery obstruction, cardiovascular diseases, inflammatory diseases, allergic diseases, thrombosis, encephalopathy, hyperlipemia, and obesity. The PDE inhibitor of the present invention has very low toxicity and thus offers a high degree of safety.

### DESCRIPTION OF THE PREFERRED EMBODIMENTS

Astaxanthin or ester thereof used in the present invention is a carotenoid represented by the following formula: 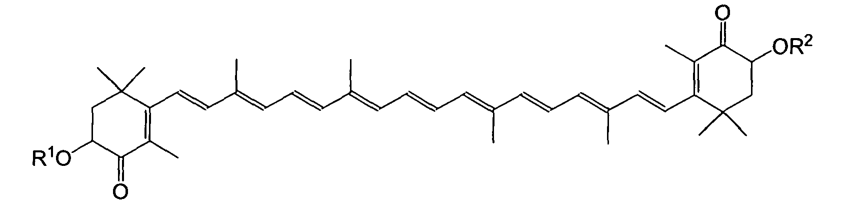 wherein R¹ and R² are both hydrogen in the case of astaxanthin, and R¹ and R² are each independently a hydrogen atom or a fatty acid residue provided that at least one of R¹ and R² is a fatty acid residue in the case of an ester of astaxanthin. Examples of the fatty acid residue forming the ester of astaxanthin include, but are not limited to, saturated fatty acids such as palmitic acid and stearic acid or unsaturated fatty acids such as oleic acid, linoleic acid, α-linolenic acid, γ-linolenic acid, bishomo-γ-linolenic acid, arachidonic acid, eicosapentaenoic acid, and docosahexaenoic acid. The astaxanthin ester of the present invention can be any mono⁻ or diester, homogeneous or non-homogeneous. Astaxanthin has a structure in which an additional oxo group and an additional hydroxy group are present at each end of a β-carotene molecule, so that unlike for β-carotene, the stability of the astaxanthin molecule is low. On the other hand, an ester form (e.g., as obtained in an extract from krill) in which the hydroxy groups at both ends are esterified with an unsaturated fatty acid is more stable.

Astaxanthin or an ester thereof used in the present invention may be chemically synthesized or derived from a naturally-occurring product. Examples of the naturally-occurring products in the latter case include red yeast; the shell of crustaceans such as Tigriopus (red water flea) and krills; and microalgae such as green algae, which contain astaxanthin and/or an ester thereof. In the present invention, any extract containing astaxanthin and/or esters thereof produced by any method can be used. Generally, extracts from those naturally⁻ occurring products can be used, and the extracts may be in the form of extracted essence, or may be appropriately purified as necessary. In the present invention, a crude extract or a crushed powder of naturally-occurring products, or a product appropriately purified or a product chemically synthesized, if necessary, that contains such astaxanthin and/or esters thereof can be used either alone or in combination as appropriate. In view of the chemical stability, an ester form of astaxanthin is preferably used.

The PDE inhibitor of the present invention may be useful for treating or preventing diseases or symptoms exhibiting a relationship to PDEs. Examples of such diseases or symptoms include cardiovascular diseases including cardiomyopathy, chronic artery obstruction, and cardiac insufficiency; pulmonary hypertension and asthma; chronic obstructive pulmonary diseases such as obliterative bronchitis, pulmonary emphysema, and bronchial asthma; atopic dermatitis, allergic rhinoconjunctivitis, autoimmune diabetes, and autoimmune encephalomyelitis; osteoarthritis and rheumatoid arthritis; inflammatory diseases such as psoriasis and ulcerative colitis; cachexia; acquired immunodeficiency syndrome (AIDS); Crohn's disease; multiple sclerosis; cerebral ischemia; reperfusion injury, restenosis, and stroke; septic shock and toxic shock; cerebral malaria; allograft rejection; depression; male erectile dysfunction (MED) and female sexual dysfunction (FSD), and dysmenorrhea; premature birth; and obesity.

The therapeutic agent for a PDE-related disease of the present invention contains astaxanthin and/or esters thereof as an active component as is the case with the PDE inhibitor of the present invention described above. In particular, the therapeutic agent is useful for treating chronic obstructive pulmonary diseases, asthma, chronic artery obstruction, cardiovascular diseases, inflammatory diseases, allergic diseases, thrombosis, encephalopathy, hyperlipemia, or obesity.

The route of administration of the PDE inhibitor or the therapeutic agent for a PDE-related disease of the present invention may be either oral or parenteral. The dosage form is selected appropriately according to the route of administration. Examples thereof include parenteral solutions, infusion solutions, powders, granules, tablets, capsules, pills, enteric-coated preparations, troches, liquids for internal use, suspensions, emulsions, syrups, liquids for external use, poultices, nose drops, ear drops, eye drops, inhalants, ointments, lotions, suppositories, and enteral nutrients. These can be used either alone or in combination depending on the condition of a disease. To prepare these dosage forms, auxiliary substances usually used in the field of pharmaceutical manufacturing technology, such as excipients, binders, antiseptics, antioxidants, disintegrators, lubricants, and flavoring agents, can be used as necessary.

The dose of the PDE inhibitor or the therapeutic agent for a PDE-related disease of the present invention varies depending on the purpose of administration, the individual to be administrated (sex, age, body weight, etc.), and the severity and nature of the disease, and can be determined by a person skilled in the art. Usually, the dose for an adult in terms of free or unesterified form of astaxanthin may be 0.1 mg to 2 g, preferably 4 mg to 500 mg per day in the case of oral administration, while it may be 0.01 mg to 1 g, preferably 0.1 mg to 500 mg per day in the case of parenteral administration.

The PDE inhibitor of the present invention can be used not only as pharmaceuticals as described above, but also as the category of products regulated as "quasi-drugs", cosmetics, functional food products, nutritional supplements, foods and drinks, and other similar products. When used as quasi-drugs or cosmetics, the PDE inhibitor may be used in conjunction with various auxiliary substances usually used in the field of quasi-drugs or cosmetics, or other technologies, if necessary. Alternatively, when used as functional food products, nutritional supplements, or foods and drinks, the PDE inhibitor may be used in conjunction with additives usually used for food products, for example, sweeteners, spices, seasonings, antiseptics, preservatives, germicides, and antioxidants, if necessary. The PDE inhibitor may be used in a desired form such as the form of solution, suspension, syrup, granule, cream, paste, or jelly, or may be shaped, if necessary. The ratio of the PDE inhibitor contained in these products is not particularly limited, and can be selected appropriately according to the intended purpose, the mode of usage, and the amount of usage.

### Examples

### Preparation Example 1: Preparation of Astaxanthin Monoester

An astaxanthin monoester was prepared in the following manner. *Haematococcus pluvialis* K0084 strain was cultivated at 25°C under irradiation with light while bubbling a gas containing 3% CO₂ into the culture medium and under nutrient stress condition (i.e. nitrogen source deprivation), and then was encysted. The encysted cells were disrupted by means usually used by those skilled in the art, and an oily fraction was extracted with ethanol. The extract contained lipids such as triglyceride in addition to astaxanthins. The extract was subjected to column chromatography using a synthetic resin adsorbent to give a purified product containing astaxanthin monoesters. This purified product was analyzed by HPLC, and it was confirmed that this purified product contained an astaxanthin monoester having a molecular weight of 858 as the main component, that the purified product did not contain the free form of astaxanthin and the diester form of astaxanthin, and that it contained a small amount of diglyceride.

### Example 1: Effect of Astaxanthin Monoester on Activity of PDE3

For the astaxanthin monoester obtained in Preparation Example 1, the effect on the activity of PDE3 was examined. PDE3 derived from human platelets was used. The astaxanthin monoester was dissolved in dimethylsulfoxide (DMSO), and the resultant solution was added to an assay system shown in Table 1 below so that the concentration was 250, 25, 2.5, and 0.25 µM, and incubated, and then, the PDE3 activity was measured to calculate the 50% inhibitory concentration and the inhibition rate.

**Table 1**

| | |
|---|---|
| Substrate | 1.01 µM([³H]cAMP + cAMP) |
| Vehicle | 1% DMSO |
| Preincubation time/ temperature | 15 minutes at 25°C |
| Incubation time/ temperature | 20 minutes at 25°C |
| Incubation buffer | 50 mM Tris-HCl, pH 7.5, 5 mM MgCl₂ |
| Quantitation method | Quantitation of [³H]adenosine |

The 50% inhibitory concentration (IC₅₀) of the astaxanthin monoester was 71.1 µM. The rate of inhibition of the enzyme activity at 250 µM was 72%, and the inhibition rate at 25 µM was 31%. Thus, the astaxanthin monoester exhibited a relatively low inhibitory concentration and a relatively high inhibition rate with respect to the PDE3 activity.

### Example 2: Effect of Astaxanthin Monoester on Activity of PDE4

For the astaxanthin monoester obtained in Preparation Example 1, the effect on the activity of PDE4 was examined, PDE4 derived from human U937 cells was used. The astaxanthin monoester was dissolved in dimethylsulfoxide (DMSO), and the resultant solution was added to the assay system shown in Table 1 above so that the concentration was 250, 25, 2.5, and 0.25 µM, and incubated, and then, the PDE4 activity was measured to calculate the 50% inhibitory concentration and the inhibition rate.

The 50% inhibitory concentration (IC₅₀) of the astaxanthin monoester was 134 µM. The rate of inhibition of the enzyme activity at 250 µM was 70%. Thus, the astaxanthin monoester exhibited a relatively low inhibitory concentration and a relatively high inhibition rate with respect to the PDE4 activity.

### Reference Example 1: Measurement of 50% Lethal Concentration for HUVEC

Human umbilical vein endothelial cells (HUVECs) (ATCC CRL-1730) were obtained from American Type Culture Collection and precultivated in an Endothelial Cell Growth Medium (CELL APPLICATIONS, USA) containing 10% bovine fetal serum supplemented with 1% Antibiotic-Antimycotic (GIBCO BRL, USA) under a 5% CO₂ atmosphere at 37°C.

A Matrigel matrix (BD Biosciences, USA) was melted and kept at 4°C on ice, and then, 50 µL of the matrix were transferred to each well of a 96-well tissue culture plate. The plate was incubated at 37°C for at least one hour to solidify the matrix solution.

On the other hand, the astaxanthin monoester obtained in Preparation Example 1 was dissolved in DMSO, and then diluted with distilled water to prepare stock test solutions in which the astaxanthin monoester was contained in 40 (v/v)% DMSO at 25000, 2500, 250, 25, and 2.5 µM, respectively.

Next, 100 µL of a HUVEC suspension (about 2.5 x 10³ cells/well) were poured into the 96-well Matrigel plate under a 5% CO₂ atmosphere at 37°C. After 24 hours, 100 µL of a growth medium and 2 µL of each of the stock test solutions or the vehicle (40 (v/v)% DMSO) were added to two wells each, and incubated for an additional 72 hours. The final concentrations of the astaxanthin monoester were 250, 25, 2.5, 0.25, and 0.025 µM, respectively.

After the incubation, 20 µL of a 90% alamarBlue reagent were added to individual wells, and incubated for an additional 6 hours. Then, the fluorescence intensity of each well was measured at an excitation wavelength of 530 nm and an emission wavelength of 590 nm using a Spectrafluor Plus plate reader to count the number of living cells. This measurement is based on the ability of a living cell to change alamarBlue from the non-fluorescent, oxidized form (blue) to the fluorescent, reduced form (red). The 50% lethal concentration was calculated when the number of living cells was 50% of the number of cells at the start of the experiment.

It was found that the 50% lethal concentration (LC₅₀) of the astaxanthin monoester for the HUVECs was 250 µM (maximum concentration of the astaxanthin monoester dissolved in DMSO) or more, and thus the toxicity of the astaxanthin monoester is low.

According to the present invention, a novel PDE inhibitor is provided. This PDE inhibitor can be used as therapeutic agents for various diseases exhibiting a relationship to PDEs, such as chronic obstructive pulmonary diseases, asthma, chronic artery obstruction, cardiovascular diseases, inflammatory diseases, allergic diseases, thrombosis, encephalopathy, hyperlipemia, and obesity. Astaxanthin and/or an ester thereof used in the present invention have been eaten for a long time and have low toxicity, and therefore have a very high degree of safety. Accordingly, the PDE inhibitor of the present invention is not only used as pharmaceuticals, but also can be used prophylactically on a daily basis as health food products.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A phosphodiesterase inhibitor comprising at least one member selected from the group consisting of astaxanthin and esters thereof.

2. A therapeutic agent for a phosphodiesterase-related disease, which comprises at least one member selected from the group consisting of astaxanthin and esters thereof.

3. The therapeutic agent of claim 2, wherein the disease is a chronic obstructive pulmonary disease, asthma, chronic artery obstruction, a cardiovascular disease, an inflammatory disease, an allergic disease, thrombosis, encephalopathy, hyperlipemia, or obesity.

4. Use of at least one member selected from the group consisting of astaxanthin and esters thereof for inhibiting a phosphodiesterase.

5. Use of at least one member selected from the group consisting of astaxanthin and esters thereof for preparing a therapeutic agent for treating a phosphodiesterase-related disease.
